# EUROPEAN PATENT SPECIFICATION

(11) **EP 1 933 143 B1**
(45) Date of publication and mention of the grant of the patent: **17.08.2011**
(21) Application number: 06025526.2
(22) Date of filing: 11.12.2006
(51) Int. Cl.: G01N 33/558, G01N 33/543, G01N 33/58, C12Q 1/68

(54) **Rapid immunochromatographic detection by amplification of the colloidal gold signal**
Schnelle immunochromatographische Detektion durch Verstärkung des kolloidalen Gold Signals
Détection rapide immunochromatographique par amplification du signal d'or colloïdal

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed Abedel-qader, Mohammed, 11118 Amman (JO)
(74) Representative: Winkler, Andreas Fritz Ernst

(56) References cited:
- EP-A- 0 590 695
- WO-A-2006/039542
- GB-A- 2 284 479
- US-A1- 2005 164 405
- TANAKA R ET AL: "A novel enhancement assay for immunochromatographic test strips using gold nanoparticles." ANALYTICAL AND BIOANALYTICAL CHEMISTRY, vol. 385, no. 8, August 2006 (2006-08), pages 1414-1420, XP002424110
- WANG ET AL: "Development of an immunochromatographic test to detect White Spot Syndrome Virus of shrimp" AQUACULTURE, vol. 255, no. 1-4, 31 May 2006 (2006-05-31), pages 196-200, XP005464138

## Description

The present invention relates to a new method for rapid immunochromatographic detection. More precisely, the present invention relates to a method for rapid immunochromatographic detection of a target in a sample, wherein the target is an antibody and/or an antigen, using double sandwich immunoassay detection for sensitivity enhancement by signal amplification. The present invention further refers to rapid immunochromatographic detection device, to uses of the method for detecting diseases or specific conditions, and to a method for the manufacture of the device as well as to a kit which comprises the device.

### BACKGROUND OF THE INVENTION

In recent years, the *in vitro* diagnostics (IVD) industry has made enormous efforts to develop immunochromatographic tests. Such tests have found applications in both clinical and non-clinical fields (1). A clinical utility of this test format has been shown for more than 150 different analytes, and many of them are target now of commercially available diagnostic products (3). The wide range of applications for such devices has been reviewed (1, 2).

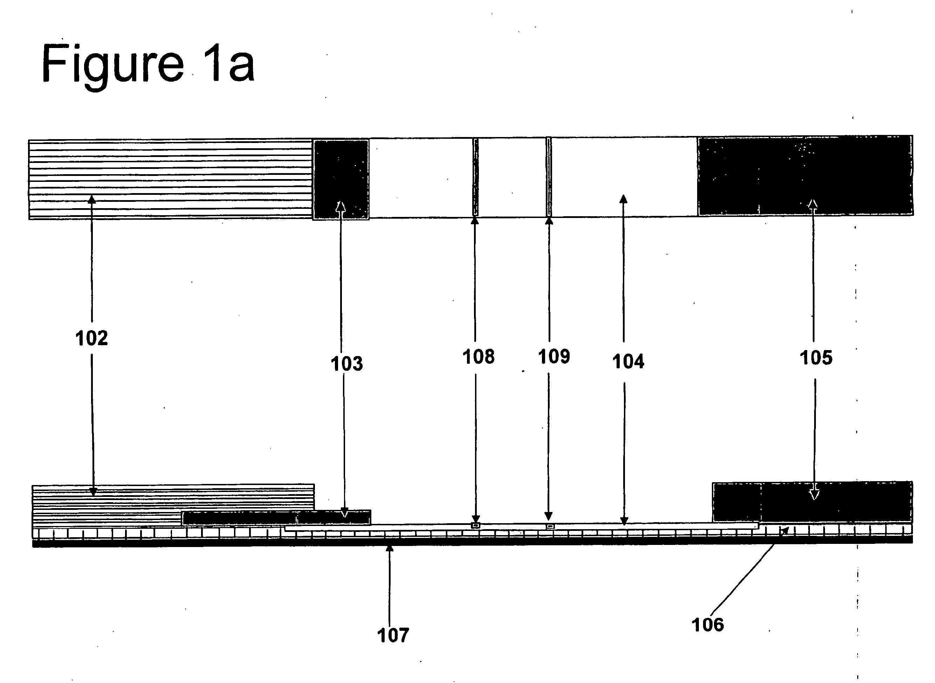

Rapid immunochromatographic test devices, e.g. in the form of a test strip, are made up of a number of components, see Figure 1a. Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispenses a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

However, despite the wide use of rapid immunochromatographic test devices, their suitability is still limited with regard to certain applications. Urine, for example, contains very low levels of IgG, frequently around 1 mg/l. Therefore, the detection of antibodies, e.g. directed to HIV or HCV, require very sensitive techniques. To date, the tests for antibodies in urine samples are based on ELISA and Western blot techniques, which are labour-intensive, time-consuming and need to be carried out by qualified persons. Efforts are being made to develop simple and/or rapid tests for the detection of antibody to HIV in urine specimens (4).

Oral fluid specimens consist often of saliva, which predominantly contains IgA class antibody, and oral mucosal transudates, which mostly contain IgG, and therefore also have much lower levels of IgG than serum. The levels of IgG normally found in oral fluid specimens (approximately 15 mg/l) are, however, higher than in urine specimens and innovative simple and rapid technology that has been shown to be effective for whole blood, serum and plasma, e.g. lateral flow through a chromatographic membrane, has been developed for use with these specimens (4).

Human chorionic gonadotropin (hCG) is a glycopeptide hormone produced by the placenta during pregnancy. The appearance and rapid increase in the concentration of hCG in the subject's urine makes it a good marker for confirming pregnancy. The concentration of hCG in urine increases steadily to a circulation peak of as much as 50,000 mIU/ml between the eighth and eleventh weeks.

Urine hCG levels during pregnancy are estimated to be:
1. 10-30 mIU/ml 7-10 days post conception.
2. 37,000-50,000 mIU/ml 8-11 weeks after last menstrual period.
3. <5 mIU/ml for healthy men or non-pregnant women.

In the prior art the hCG test is a chromatographic immunoassay which uses specific antibodies to selectively identify hCG in urine with a high degree of sensitivity. Elevated levels of hCG as low as 20 mIU/ml can be detected within 3 minutes.

There are several tests used to detect the presence of hepatitis B antibodies. There are also several tests that detect the presence of viral antigens. The hepatitis B surface antibody (anti-HBs) is the most common test. Its presence indicates previous exposure to HBV, but the virus is no longer present and the person cannot pass on the virus to others. The antibody also protects the body from future HBV infection. In addition to exposure to HBV, the antibodies can also be acquired from successful vaccination. This test is done to determine the need for vaccination (if anti-HBs is absent), or following the completion of vaccination against the disease, or following an active infection.

Hepatitis B surface antigen (HBsAg) is a protein antigen produced by HBV. This antigen is the earliest indicator of acute hepatitis B and frequently identifies infected people before symptoms appear. HBsAg disappears from the blood during the recovery period. In some people (particularly those infected as children or those with a weak immune system, such as those with AIDS), chronic infection with HBV may occur and HBsAg remains positive. To test for HIV is an essential component in the diagnosis and treatment of persons infected with the virus, in screening of blood for transfusion, in surveillance and in HIV/AIDS related research. Thus accurate and cost-effective testing is of great importance in combating the spread of HIV. It is imperative that tests for the diagnosis of HIV infection be as accurate as possible, given the serious ethical, legal and social issues that accompany HIV infection.

The number of people living with HIV has now risen to reach its highest level ever: close to 40 million people are living with the virus and close to 5 million people were newly infected with HIV in 2004 alone. Worldwide, the AIDS epidemic killed over 3 million people last year alone (Source: UNAIDS). Furthermore, only one in five people needing HIV prevention worldwide have access to basic prevention services and only one in ten people living with HIV has been tested for the virus.

The HI virus is most easily transmitted to others during the initial period of acute HIV infection, when the viral load (quantity of HIV RNA in the blood) is especially high and when people are not aware of being contaminated by the virus. Most HIV infections are transmitted at this stage, called primary infection. Earlier detection using ultra sensitive tests avoids missing primary infections, enabling immediate precautionary measures to be taken to help prevent the risk of HIV transmission to a non-infected partner, to an unborn child, or through blood donations or direct blood contact. Earlier detection of HIV infection also ensures the implementation of early antiretroviral therapy (ART) to slow down the progression of HIV infection, thereby improving patient care and quality of life.

The diagnosis of HIV infection is usually made on the basis of the detection of HIV antibodies and/or antigen. The diagnosis of an HIV infection can be made indirectly, i.e. through the demonstration of virus-specific antibodies. Besides such indirect diagnosis based on detection of antibodies, a direct diagnosis of HIV infection is also possible: either through the demonstration of infectious virus (using cell culture), viral antigens (p24 antigen ELISA) or viral nucleic acid (i.e. viral genome); the latter is also termed nucleic acid testing (NAT).

One important problem of HIV antibody testing is the so-called "diagnostic window". This is the time period that elapses between the time of acquisition of HIV infection until detectable levels of antibodies are present. The switch from antibody-negative to antibody-positive is called "seroconversion".

The most widely used screening tests are ELISAs as they are the most appropriate for screening large numbers of specimens on a daily basis, e.g. blood donations. The earliest assays used purified HIV lysates (1st generation assays). Improved assays based on recombinant proteins and/or synthetic peptides, which also enabled the production of combined HIV-1/HIV-2 assays, became rapidly available (2nd generation assays). The so-called 3rd generation or antigen-sandwich assays, which use labeled antigens as conjugate, are more sensitive and have reduced the diagnostic window period considerably (5, 6).

Tanaka *et al.* 2006 (7) refers to a highly sensitive immunochromatographic assay applied to detect hCG as the model case. Primary antibody-conjugated gold nanoparticles were used as an enhancer. The primary antibodies were immobilized within a defined detection zone (test line) on the diagnostic nitrocellulose membrane. Secondary antibodies were conjugated with colloidal gold nanoparticles. The gold-conjugated antibodies and the primary antibodies formed a sandwich complex with the target protein. Within the test line, the sandwich complex was immobilized.

US 2005/164405 refers to a chromatographic immunoassay test strip in which an analyte in a sample reacts with a ligand to form a "ligand A-analyte-ligand B/tracer" first, then is captured by a bridge immobilized on the test zone of a solid phase to form a complex of "bridge-ligand A-analyte-ligand B/tracer" which can then be detected.

Wang *et al.* (2006) (8) refers to an immunochromatographic test device for detecting white spot syndrome virus of shrimp using monoclonal antibodies labeled with colloidal gold as a detection reagent and a capture antibody immobilized on a nitrocellulose membrane.

EP 0 590 695 refers to a liquid transfer device for use in assay procedures comprising a sheet of porous material for capillary liquid flow therethrough.

GB 2 284 479 refers to a liquid transfer device having utility in diagnostic assays comprising first and second capillary flow channels.

WO 2006/039542 refers to an analytical device for performing an assay to determine the presence or approximate quantity of an analyte in a liquid sample, the device comprising primary and secondary flow paths and a capture zone.

It is an object of the present invention to overcome the problems with regard to the applicability of rapid immunochromatographic test devices for the detection of hCG, HBsAG, anti-HBs, IgG, e.g. HIV antibodies, in urine, blood, serum or saliva by enhanced sensitivity. Therefore, it is an object to enhance the sensitivity of the rapid immunochromatographic detection system by the improvement of the colloidal gold signal.

### SUMMARY OF THE PRESENT INVENTION

It is the object of the present invention to solve the rapid immunochromatographic detection of a target in a sample by a new method through sensitivity enhancement using different colloidal gold conjugates conjugated with a first and a second specific antibody or antigen, respectively. The rapid immunochromatographic detection method is using the double sandwich immunoassay detection to multiply the colloidal gold signal system comprising a detection test strip of two gold conjugate releasing pads with different compositions.

In one embodiment the present invention concerns a method for rapid immunochromatographic detection of a target in a sample comprising the step of forming a double sandwich by contacting the sample with
(a) a first colloidal gold conjugate conjugated with a first specific antibody or specific antigen to capture the target from a first site A; followed by
(b) a second specific antibody or specific antigen to capture the target from a second site B, wherein the second specific antibody or specific antigen is immobilized; and followed by
(c) a second colloidal gold conjugate conjugated with the second specific antibody or specific antigen that is the same specific antibody or specific antigen as is immoblized.

Thus, this is achieved by sensitivity enhancement using different colloidal gold conjugates conjugated with a first and a second specific antibody or antigen, respectively. The rapid immunochromatographic detection method is using the double sandwich immunoassay detection to multiply the colloidal gold signal system comprising a detection test strip of two gold conjugate releasing pads with different compositions.

In one embodiment the present invention further relates to a method, comprising the following additional steps of
(d) applying the sample to a sample application site,
(e) allowing the target in the sample getting captured from the first target site A by the first colloidal gold conjugate from a first conjugate releasing site,
(f) allowing the target in the sample to move to a test zone for being captured from the second target site B by the immobilized second specific antibody or specific antigen ,
(g) allowing to release the second colloidal gold conjugate from a second conjugate releasing site for capturing the target in the sample from the second target site B,
(h) allowing the sample to move through the test zone and the control zone to an absorbent site,
(i) allowing to continuously release the first and the second colloidal gold conjugates from the first and second conjugate releasing sites to propagate to the test zone and the control zone,
(j) detecting a color in the control zone, and
(k) detecting a color in the test zone.

In a further embodiment the present invention concerns a test device for conducting the method for rapid immunochromatographic detection of a target in a sample according to the present invention comprising a housing comprising a test strip comprising
(a) a sample application site (102);
(b) a first conjugate releasing site (103.1) comprising a first colloidal gold conjugate conjugated with a first specific antibody or specific antigen to capture the target from a first site A;
(c) a second conjugate releasing site (103.2) comprising a second colloidal gold conjugate conjugated with a second specific antibody or specific antigen to capture the target from the second site B;
   wherein the first and the second conjugate releasing sites are separated by a divider or wherein the first conjugate releasing site is on the test strip and the second conjugate releasing site is within the upper side of the housing;
(d) a nitrocellulose membrane (104);
(e) a test zone (108) comprising the second specific antibody or specific antigen immobilized;
(f) a control zone (109); and
(g) a sample absorbent site (105).

In another embodiment the present invention relates to the use of the method for diagnosing and monitoring a disease or a specific condition of a subject by detecting a target in a sample.

In a further embodiment the present invention refers to a kit for rapid immunochromatographic detection of a target in a sample comprising the test device according to the present invention.

In a further embodiment the present invention concerns a method for the manufacture of the test device according to the present invention comprising the following steps of
(a) preparing a first colloidal gold conjugate by adding a first specific antibody or antigen to a conjugation buffer and then adding it to a colloidal gold solution,
(b) preparing a second colloidal gold conjugate by adding a second specific antibody or antigen to a conjugation buffer and then adding it to a colloidal gold solution, and
(c) preparing a first conjugate releasing site and a second conjugate releasing site by applying the first and the second gold conjugate on different pads,
   wherein the first and the second conjugate releasing sites are separated by a divider or wherein the first conjugate releasing site is on the test strip and the second conjugate releasing site is within the upper site of a housing.

In another embodiment the method for the manufacture of the test device according to the present invention comprises the following additional steps of
(d) preparing a sample application site 102, a test zone 108, a control zone 109 and a sample absorbent site 105,
(e) assembling the sample application site 102, the test zone 108, the control zone 109, the sample absorbent site 105 together with the first 103.1 and the second conjugate releasing sites on a test strip 101,
(f) applying the first and the second colloidal gold conjugates on different sites of the same card separated by the divider 110, and
(g) assembling the test strip 101 in a housing.

In a further embodiment the method for the manufacture of the test device according to the present invention comprises the following additional steps of
(d) preparing a sample application site 102, a test zone 108, a control zone 109 and a sample absorbent site 105,
(e) assembling the sample application site 102, the test zone 108, the control zone 109, the sample absorbent site 105 together with the first conjugate releasing site 103.1 on a test strip 101, and
(f) assembling the test strip 101 and the second conjugate releasing site 103.2 in a housing.

The first conjugate releasing site of the rapid immunochromatographic detection system contains a colloidal gold conjugate that is conjugated with the first specific antibody or antigen to capture the target from the first site (let us consider it as site A), while the second conjugate releasing site contains a colloidal gold conjugate conjugated with the second specific antibody or antigen to capture the target from the second site (let us consider it as site B). The last mentioned conjugated antibody or antigen is the same antibody or antigen that is immobilized onto the nitrocellulose membrane.

By the sample flow within the rapid immunochromatographic test the antibody on the first conjugate releasing site will capture the first specific antigen or antibody in the sample from site A and carry it to be captured by the second specific antibody or antigen that is immobilized onto the nitrocellulose membrane from site B to form the sandwich detection. Taking in mind that the first conjugate mostly capture more than one target molecule, one ore more targets will be captured from A and will be free from site B.

Then, when the second conjugate releasing site releases its colloidal gold conjugate that is conjugated with the same antibody or antigen that is immobilized onto the nitrocellulose membrane, this conjugate will capture site B of the already captured targets by the first conjugate which is captured by the antibody or antigen onto the membrane.

The new binding of the second conjugate will make new probability choices of capturing the unbound target or captured target (by the first conjugate), similar for the unbound first conjugate, to form more and more branched bonds that propagate the accumulation of colloidal gold particles onto the test zone. This propagation and accumulation of colloidal gold signal will improve the signal and increase the sensitivity.

### DETAILED DESCRIPTION OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms : (IUPAC Recommendations)", Leuenberger, H.G.W., Nagel, B. and Kölbl. H. eds. (1996), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step.

As outlined above there is a need in the prior art to provide a new method for rapid immunochromatographic detection of a target in a sample for the detection of a disease or a specific condition such as pregnancy in a subject. There is also a need in the art for methods suitable for rapid and sensitive detection of an antibody and/or antigen having a higher sensitivity than methods from the prior art.

In a first aspect the present invention provides a method for rapid immunochromatographic detection of a target in a sample comprising the step of forming a double sandwich by contacting the sample with
(a) a first colloidal gold conjugate conjugated with a first specific antibody or specific antigen to capture the target from a first site A; followed by
(b) a second specific antibody or specific antigen to capture the target from a second site B, wherein the second specific antibody or specific antigen is immobilized; and followed by
(c) a second colloidal gold conjugate conjugated with the second specific antibody or specific antigen that is the same specific antibody or specific antigen as is immoblized.

The first colloidal gold conjugated with a first antibody or antigen captures the target in the sample and forms a complex "target-first colloidal conjugate". Preferably this target in the sample is an antigen and/or antibody.

In a further first aspect the present invention provides a method comprising the following additional steps of
(d) applying the sample to a sample application site,
(e) allowing the target in the sample getting captured from the first target site A by the first colloidal gold conjugate from a first conjugate releasing site.
(f) allowing the target in the sample to move to a test zone for being captured from the second target site B by the immobilized second specific antibody or specific antigen,
(g) allowing to release the second colloidal gold conjugate from a second conjugate releasing site for capturing the target in the sample-from the second target site B,
(h) allowing the sample to move through the test zone and the control zone to an absorbent site,
(i) allowing to continuously release the first and the second colloidal gold conjugates from the first and second conjugate releasing sites to propagate to the test zone and the control zone,
(j) detecting a color in the control zone, and
(k) detecting a color in the test zone.

In one embodiment the method comprises further a specific first antibody or antigen which is selected from the group consisting of anti-beta chorionic gonadotropin hormone (anti-βhCG), anti-lipoarabinomannan (LAM), hepatitis virus antibodies against or antigens from hepatitis virus type A , hepatitis virus type B, or hepatitis virus type C or human immunoglobulin G antibodies or antigens.

Other antigens or antibodies that could be used for related antibody detection are: H.Pylori antigen, hepatitis B envelope antigen, hepatitis C NS3 antigen, hepatitis C core antigen, HIV p160, HIV p24, Toxoplasma antigen, anti-Malaria HRP-II, anti-human immunoglobulin G, anti- LH, anti-prostate specific antigen, anti-pneumolysin, anti-leishmania antigen, anti-H.Pylori antigen, and anti-toxoplasma antigen. Monoclonal antibodies are preferred while polyclonal antibodies are applicable.

In one preferred embodiment the hepatitis virus antigen is hepatitis B surface antigen (HbsAg) and the hepatitis virus antibody is anti-HBsAg.

In one embodiment the method comprises a second specific antibody or antigen which is selected from the group consisting of anti-alpha chorionic gonadotropin hormone (anti-αhCG), anti-lipoarabinomannan (LAM), hepatitis virus antibodies against or antigens from hepatitis virus type A, hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

In one preferred embodiment the hepatitis virus antigen is is hepatitis B surface antigen (HbsAg), the hepatitis virus antibody is anti-HBsAg and the human immunodeficiency virus (HIV) antigen is HIV p 160.
Other antigens or antibodies that could be used for related antibody detection are: H.Pylori antigen, hepatitis B envelope antigen, hepatitis C NS3 antigen, hepatitis C core antigen, HIV p160, HIV p24, Toxoplasma antigen, anti-Malaria HRP-II, anti-human immunoglobulin G, anti- LH, anti-prostate specific antigen, anti-pneumolysin, anti-leishmania antigen, anti-H.Pylori antigen, and anti-toxoplasma antigen. Monoclonal antibodies are preferred while polyclonal antibodies are applicable.

In one embodiment of the method the sample comprises a body fluid obtained from a subject.

In one preferred embodiment the body fluid is selected from the group consisting of urine, whole blood, serum plasma and saliva.

In another aspect the present invention concerns a test device for conducting the method for rapid immunochromatographic detection of a target in a sample according to the present invention comprising a housing comprising a test strip 101 comprising
(a) a sample application site (102);
(b) a first conjugate releasing site (103.1) comprising a first colloidal gold conjugate conjugated with a first specific antibody or specific antigen to capture the target from a first site A;
(c) a second conjugate releasing site (103.2) comprising a second colloidal gold conjugate conjugated with a second specific antibody or specific antigen to capture the target from the second site B;
   wherein the first and the second conjugate releasing sites are separated by a divider or wherein the first conjugate releasing site is on the test strip and the second conjugate releasing site is within the upper side of the housing;
(d) a nitrocellulose membrane (104);
(e) a test zone (108) comprising the second specific antibody or specific antigen immobilized;
(f) a control zone (109); and
(g) a sample absorbent site (105).

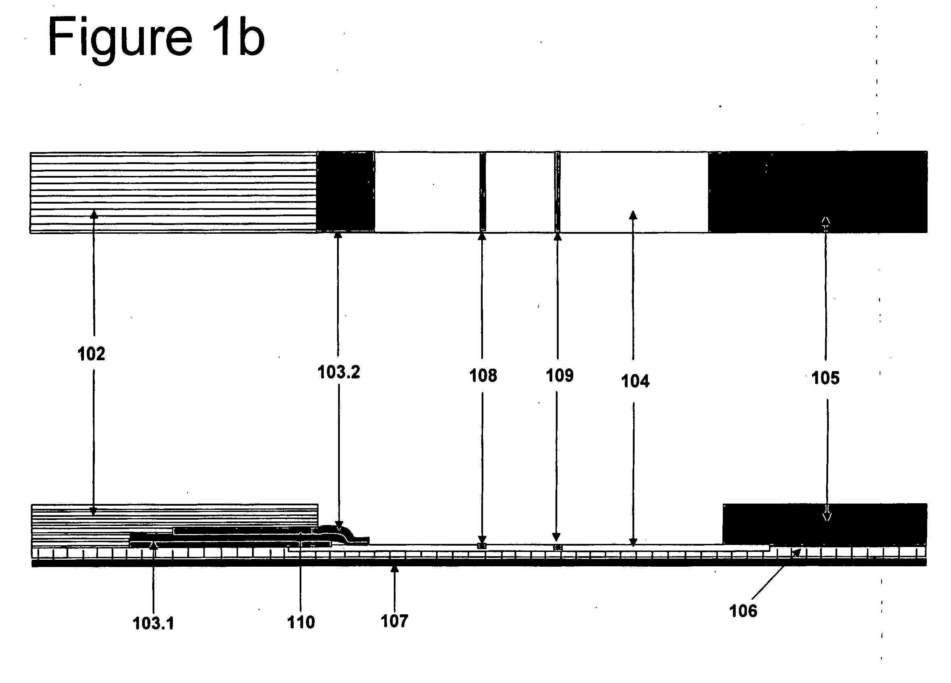

The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see Figure 1b. The second conjugate releasing site can be laminated within the upper side of the plastic housing of the device.

In one embodiment the test device further comprises the test strip 101 which is attached to a supporting backing 107 by means of an adhesive 106.

In a preferred embodiment the supporting backing 107 of the test device is a plastic backing.

In another embodiment the test zone 108 of the test device comprises the second specific antibody or antigen.

In another preferred embodiment the test zone 108 of the test device comprises the second specific antibody or antigen which is selected from the group consisting of anti-alpha chorionic gonadotropin hormone (anti-αhCG), anti-lipoarabiniomannan (LAM), hepatitis virus antibodies against or antigens from hepatitis virus type A, hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

In a more preferred embodiment the hepatitis virus antigen is hepatitis B surface antigen (HBsAg), the hepatitis virus antibody is anti-HBsAg and the human immunodeficiency virus (HIV) antigen is HIV p160.

In another embodiment the second conjugate releasing site 103.2 of the test device is laminated within the upper side of the housing.

In another aspect the invention relates to the use of the method for diagnosing and monitoring a disease or a specific condition of a subject by detecting a target in a sample.

In one embodiment the specific condition is pregnancy.

In a preferred embodiment the target of the specific condition is human chorionic gonadotropin hormone (hCG).

In another embodiment the disease is hepatitis selected of the group consisting of hepatitis type A, hepatitis type B, or hepatitis type C.

In a preferred embodiment the selected hepatitis type is hepatitis type B.

In a more preferred embodiment the target of the disease which is hepatitis type is hepatitis B surface antigen (HbsAg).

In another embodiment the disease is an HIV infection selected from the HIV infection group consisting of HIV type HIV-1 and HIV-2 or HIV subtype HIV-1-N, HIV-1-O or HIV-1-M.

In a more preferred embodiment the target of the HIV infection is selected from an HIV antibody or antigen selected from the group consisting of p41, p120, p160, p18, p24/25, p55, p34, p40, p52, p68.

In a further more preferred embodiment the HIV antigen is p160.

In a further aspect the invention concerns a kit for rapid immunochromatographic detection of a target in a sample comprising the test device comprising the housing according to the invention.

In one embodiment the kit comprises further reagents, wash buffers and a manual.

There is no need for special reagents or buffers except in the case of a whole blood sample, where the used assay buffer is 0.1M Tris buffer (pH 7.5) with sodium azide 0.01M as a preservative.

In another aspect the invention relates to a method for the manufacture of the test device according to the invention

comprising the following steps of
(a) preparing a first colloidal gold conjugate by adding a first specific antibody or specific antigen to a conjugation buffer and then adding it to a colloidal gold solution,
(b) preparing a second colloidal gold conjugate by adding a second specific antibody or specific antigen to a conjugation buffer and then adding it to a colloidal gold solution, and
(c) preparing a first conjugate releasing site and a second conjugate releasing site by applying the first and the second colloidal gold conjugate on different pads, wherein the first and the second conjugate releasing sites are separated by a divider or wherein the first conjugate releasing site is on the test strip and the second conjugate releasing site is within the upper site of a housing.

In one embodiment the method further comprises the following steps of
(d) preparing a sample application site 102, a test zone 108, a control zone 109 and a sample absorbent site 105,
(e) assembling the sample application site 102, the test zone 108, the control zone 109, the sample absorbent site 105 together with the first 103.1 and the second conjugate releasing sites on a test strip 101,
(f) applying the first and the second colloidal gold conjugates on different sites of the same card separated by the divider 110, and
(g) assembling the test strip 101 in a housing.

In anther embodiment the method further comprises the following steps of
(d) preparing a sample application site 102, a test zone 108, a control zone 109 and a sample absorbent site 105,
(e) assembling the sample application site 102, the test zone 108, the control zone 109, the sample absorbent site 105 together with the first conjugate releasing site 103.1 on a test strip 101, and
(f) assembling the test strip 101 and the second conjugate releasing site 103.2 in a housing.

### BRIEF DESCRIPTION OF THE FIGURES

**Figure 1a****:**
   Figure 1a shows top and side views of a typical rapid-flow immunochromatographic test device in the form of a test strip 101 including a sample pad 102, a conjugate pad 103, a membrane 104, an absorbent pad 105, an adhesive 106, a supporting backing 107, a test zone 108, and a control zone 109.
**Figure 1b****:**
   Figure 1b shows top and side views of our modified rapid-flow immunochromatographic test device in the form of a test strip 101 including a sample pad 102, a first conjugate pad 103.1, a second conjugate pad 103.2, a membrane 104, an absorbent pad 105, an adhesive 106, a supporting backing 107, a test zone 108, a control zone 109, and the divider 110.
**Figure 2****:**

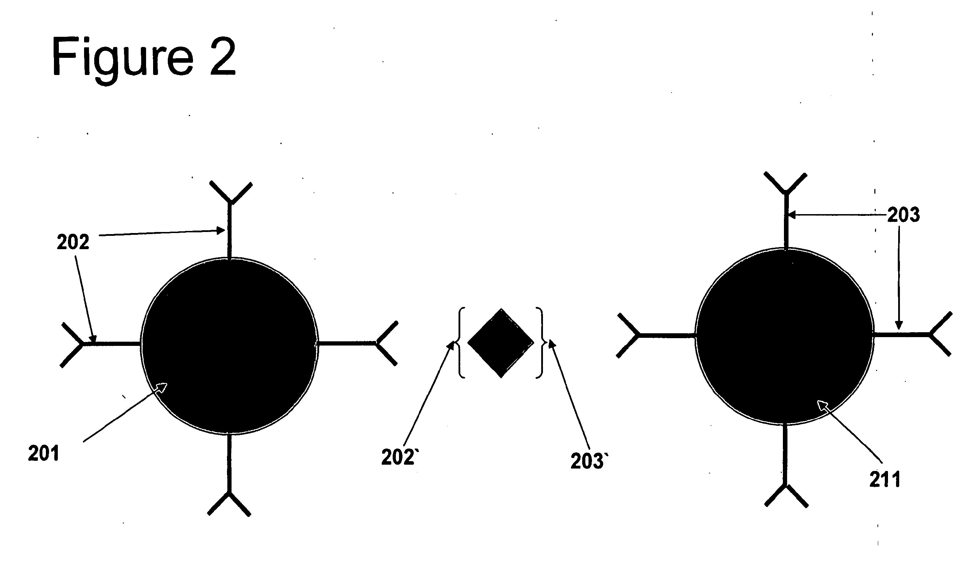

   Figure 2 shows the schematically view of the first 201 and second 211 colloidal gold, whereas the first colloidal gold 201 is conjugated with a first specific antibody 202 to capture the target at side A or antigen 202' and the second colloidal gold is conjugated with a second specific antibody or antigen 203 to capture the target at side B 203'.
**Figure 3****:**

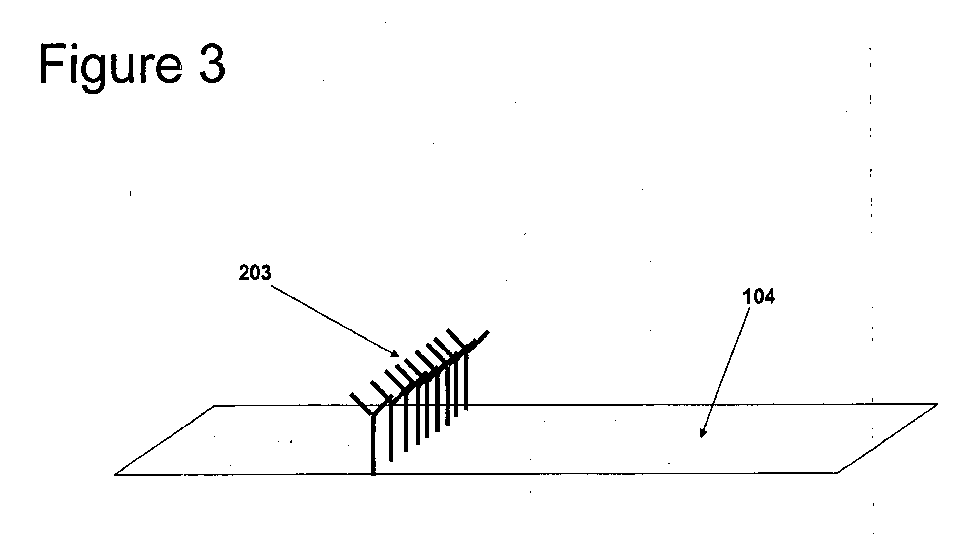

   Figure 3 shows a simplified scheme of the test zone 108 of the nitrocellulose membrane 104 of the test strip 101. The second specific antibody or antigen 203 is immobilized to the test zone 108.
**Figure 4****:**

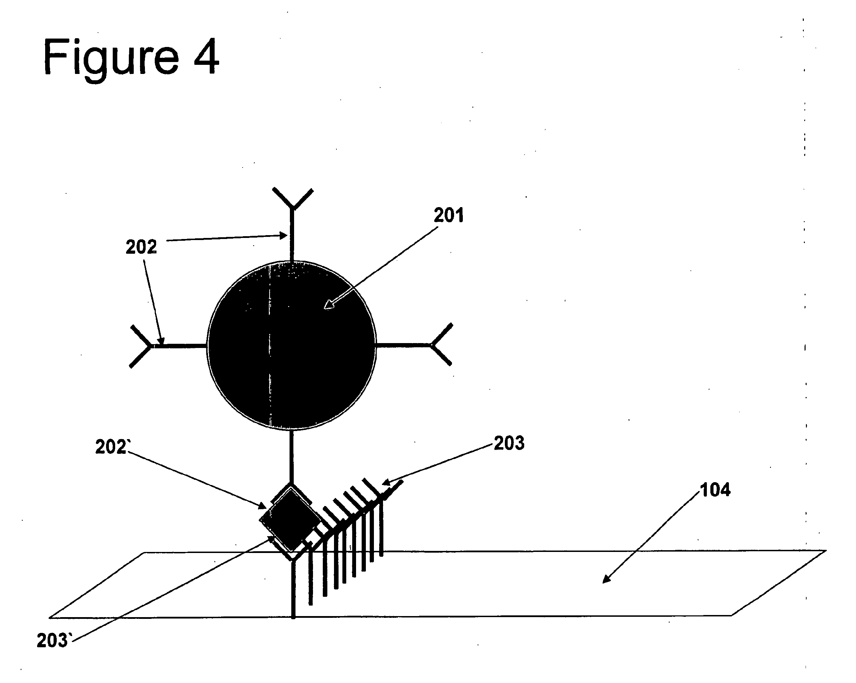

   Figure 4 shows the main principle of signal development. By the sample flow within the rapid immunochromatographic test device the target in the sample will be captured by the antibody or antigen 202 of the first colloidal gold 201 to form the complex "target-first colloidal gold". This complex flows to the test zone 108, where it will be captured by the second antibody or antigen 203 that is immobilized onto the membrane 104 of the test zone 108 to form a sandwich detection.
**Figure 5****:**

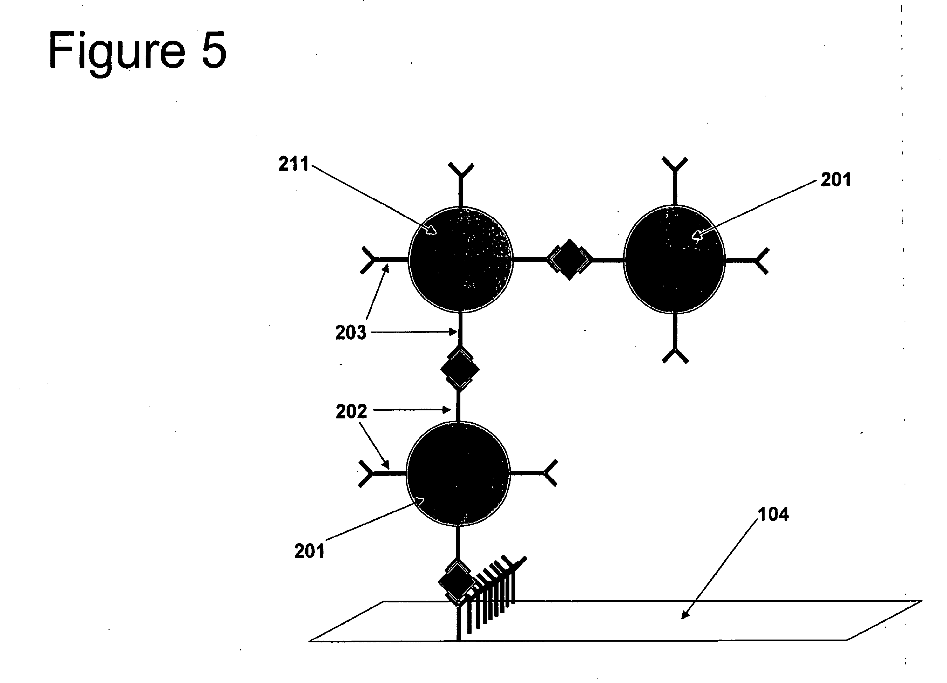

   Figure 5 shows the main principle of signal amplification:
   By the sample flow within the rapid immunochromatographic test device the target in the sample will be captured by the first specific antibody or antigen 202 of the first colloidal gold 201 to form the complex "target- first colloidal gold". This complex flows to the test zone,
   where it will be captured by the second specific antibody or antigen 203 that is immobilized onto the membrane 104 of the test zone 108. Then, the second colloidal gold 211 conjugated with the first specific antibody or antigen 203 will be released and will bind to the target as well as to the first colloidal gold conjugate 201 and enhance the signal by forming a double sandwich.

### EXAMPLES

The following examples illustrate the present invention without, however, limiting the same thereto.

### Example 1: Pregnancy detection system

The first gold conjugate is mouse anti-βhCG conjugated with colloidal gold conjugate, and the second gold conjugate is mouse anti-αhCG conjugated with colloidal gold conjugate. The first gold conjugate 103.1 was laminated in the side of the nitrocellulose membrane 104 while the second gold conjugate 103.2 was laminated above the first pad 103.1 separated by a divider 110 that enables the second conjugate to take a part of the sample and release directly onto the nitrocellulose membrane 104.

The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see Figure 1b. The second conjugate releasing site could be laminated within the upper side of the device plastic housing.

The test zone 108 is a mouse anti-αhCG immobilized onto the nitrocellulose membrane 104. The control zone 109 is anti-mouse IgG. Test 108 and control 109 zones turn into purple color in case of hCG availability in the sample; only the control zone 109 turns into purple color in case of hCG free sample.

The commercially available rapid tests sensitivity for the pregnancy hormone which is human chorionic gonadotropin hormone (hCG) is around 25mIU/ml while according to this system it is so simple to detect less than 5 mIU/ml.

### Example 2: Hepatitis B surface antigen (HBsAg) detection system

The first gold conjugate is made of mouse anti-HBsAg (clone 1) conjugated with colloidal gold conjugate, and the second gold conjugate is mouse anti-HBsAg (clone 2) conjugated with colloidal gold conjugate. The numbering of clones are only for explanation and to recognize that we use always two different clones of monoclonal antibodies; these two monoclonal antibodies capture the target antigen from two different sites, so we call them as: a pair of monoclonal antibodies. The first gold conjugate 103.1 was laminated in the side of the nitrocellulose membrane 104, while the second gold conjugate was laminated above the first pad 103.1 separated by a divider 110 that enables the second conjugate to take a part of the sample and release it directly onto the nitrocellulose membrane 104.

The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see Figure 1b. The second conjugate releasing site could be laminated within the upper side of the device plastic housing.

The test zone 108 is mouse anti-HBsAg (clone2) immobilized onto the nitrocellulose membrane 104. The control zone 109 is anti-mouse IgG. Test 108 and control zones 109 turn into purple color in case of HBsAg availability in the sample; only the control zone 109 turns into purple color in case of HBsAg free sample, see Figure1b.

The commercially available rapid tests sensitivity for hepatitis B surface antigen is within the range 500-1000pg/ml while according to this system it is so simple to detect less than 50 pg/ml.

### Example 3: Human Immunodeficiency Virus (HIV) antibodies detection system

The first gold conjugate is mouse anti-human Immunoglobulin G (anti-hIgG) conjugated with colloidal gold conjugate, and the second gold conjugate is HIV p160 conjugated with colloidal gold conjugate. The first gold conjugate 103.1 was laminated in the side of nitrocellulose membrane 104, while the second gold conjugate was laminated above the first pad 103.1 separated by a divider 110 that enables the second conjugate to take a part of the sample and release directly onto the nitrocellulose membrane 104.

The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see Figure 1b. The second conjugate releasing site could be laminated within the upper side of the device plastic housing.

The test zone 108 is HIV p160 antigen immobilized onto the nitrocellulose membrane 104. The control zone 109 is anti-mouse IgG. Sample 108 and control 109 zones turn into purple color in case of HIV antibodies availability in the sample; only the control zone 109 turns into purple color in case of HIV antibodies free sample, see Figurelb.

According to this system it is so simple to detect very low titers of HIV antibodies.

### References

(1) J Chandler, N Robinson, and K Whiting,"Handling False Signals in Gold-Based Rapid Tests", IVD Technology 7, no. 2 (2001): 3445; httn://www.devicelink.com/ivdt/archive/01/03/002.html.
(2) J Chandler, T Gurmin, and N Robinson,"The Place of Gold in Rapid Tests", IVD Technology 6, no. 2 (2000): 37-49; http://www.devicelink.com/ivdt/archive/00/03/004.html
(3) TC Tisone et al.,''Image Analysis for Rapid-Flow Diagnostics", IVD Technology 5, no. 5 (1999): 52-58; http://www.devicelink.com/ivdt/archive/99/09/010.html
(4) World Health Organization, HIV assays: operational characteristics (Phase I). Report 13: urine specimens, oral fluid (saliva) specimens. [Material originally distributed as WHO/BCT/02.08]
(5) Zaaijer, H.L., Exel-Oehlers, P.V., Kraaijeveld, T., Altena, E., Lelie, P.N. (1992) Early detection of antibodies to HIV-1 by third-generation assays. Lancet 340, 770-772.
(6) Constantine, N.T., van der Groen, G., Belsey, E.M., Tamashiro, H. (1994) Sensitivity of HIV-antibody assays determined by seroconversion panels. AIDS 8,1715-1720.
(7) R Tanaka, T Yuhi, N Nagatani, T Endo, K Kerman, Y Takamura, E Tamiya (2006) A novel enhancement assay for immunochromatographic test strips using gold nanoparticles. Anal Bioanal Chem 385: 1414-1420.
(8) X Wang and W Zhan (2006) Development of an immunochromatographic test to detect White Spot Syndrome Virus of shrimp. Aquaculture 255: 196-200.

## Claims

1. A method for rapid immunochromatographic detection of a target in a sample comprising the step of forming a double sandwich, by contacting the sample with
(a) a first colloidal gold conjugate conjugated with a first specific antibody or specific antigen to capture the target from a first site A; followed by
(b) a second specific antibody or specific antigen to capture the target from a second site B, wherein the second specific antibody or specific antigen is immobilized; and followed by
(c) a second colloidal gold conjugate conjugated with the second specific antibody or specific antigen that is the same specific antibody or specific antigen as is immoblized.

2. The method according to claim 1, comprising the following steps of
(d) applying the sample to a sample application site;
(e) allowing the target in the sample getting captured from the first target site A by the first colloidal gold conjugate from a first conjugate releasing site;
(f) allowing the target in the sample to move to a test zone for being captured from the second target site B by the immobilised second specific antibody or specific antigen ;
(g) allowing to release the second colloidal gold conjugate from a second conjugate releasing site for capturing the target in the sample from the second target site B;
(h) allowing the sample to move through the test zone and a control zone to an absorbent site;
(i) allowing to continuously release the first and the second colloidal gold conjugates from the first and second conjugate releasing sites to propagate to the test zone and the control zone;
(j) detecting a color in the control zone; and
(k) detecting a color in the test zone.

3. The method according to claims 1 or 2, wherein the first specific antibody or antigen is selected from the group consisting of anti-beta chorionic gonadotropin hormone (anti-βhCG), anti-lipoarabinomannan (LAM), hepatitis virus antibodies against or antigens from hepatitis virus type A , hepatitis virus type B, or hepatitis virus type C or human immunoglobulin G antibodies or antigens.

4. The method according to claim 3, wherein the hepatitis virus antigen is hepatitis B surface antigen (HBsAg) and the hepatitis virus antibody is anti-HBsAg.

5. The method according to any of claims 1 to 4, wherein the second specific antibody or antigen is selected from the group consisting of anti-alpha chorionic gonadotropin hormone (anti-αhCG), anti-lipoarabinomannan (LAM), hepatitis virus antibodies against or antigens from hepatitis virus type A , hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

6. The method according to claim 5, wherein the hepatitis virus antigen is hepatitis B surface antigen (HBsAg), the hepatitis virus antibody is anti-HBsAg and the human immunodeficiency virus (HIV) antigen is HIV p160.

7. The method according to any of claims 1 to 6, wherein the sample comprises a body fluid obtained from a subject.

8. The method according to claim 7, wherein the body fluid is selected from the group consisting of urine, whole blood, serum, plasma and saliva.

9. A test device for conducting the method for rapid immunochromatographic detection of a target in a sample of any of claims 1 to 8 comprising a housing comprising a test strip (101) comprising
(a) a sample application site (102);
(b) a first conjugate releasing site (103.1) comprising a first colloidal gold conjugate conjugated with a first specific antibody or specific antigen to capture the target from a first site A;
(c) a second conjugate releasing site (103.2) comprising a second colloidal gold conjugate conjugated with a second specific antibody or specific antigen to capture the target from the second site B;
wherein the first and the second conjugate releasing sites are separated by a divider or wherein the first conjugate releasing site is on the test strip and the second conjugate releasing site is within the upper side of the housing;
(d) a nitrocellulose membrane (104);
(e) a test zone (108) comprising the second specific antibody or specific antigen immobilized;
(f) a control zone (109); and
(g) a sample absorbent site (105).

10. The test device according to claim 9, wherein the test strip (101) is attached to a supporting backing (107) by means of an adhesive (106).

11. The test device according to claim 10, wherein the supporting backing (107) is a plastic backing.

12. The test device according to claim 9, 10 or 11, wherein the second specific antibody or antigen is selected from the group consisting of anti-alpha chorionic gonadotropin hormone (anti-αhCG), anti-lipoarabinomanna (LAM), hepatitis virus antibodies against or antigens from hepatitis virus type A , hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

13. The test device according to claim 12, wherein the hepatitis virus antigen is an hepatitis B surface antigen (HBsAg), the hepatitis virus antibody is anti-HBsAg and the human immunodeficiency virus (HIV) antigen is HIV p 160.

14. The test device according to any of claims 9 to 13, wherein the second conjugate releasing site (103.2) is laminated within the upper side of the housing.

15. Use of the method according to any of claims 1 to 8 for diagnosing and monitoring a disease or a specific condition of a subject by detecting a target in a sample.

16. Use according to claim 15, wherein the specific condition is pregnancy.

17. Use according to claims 15 or 16, wherein the target is human chorionic gonadotropin hormone (hCG).

18. Use according to claim 15, wherein the disease is hepatitis selected of the group consisting of hepatitis type A, hepatitis type B, or hepatitis type C.

19. Use according to claim 18, wherein the selected hepatitis type is hepatitis type B.

20. Use according to the claims 15, 18 or 19, wherein the target is hepatitis B surface antigen (HBsAg).

21. Use according to claim 15, wherein the disease is an HIV infection selected from the HIV infection group consisting of HIV type HIV-1 and HIV-2 or HIV subtype HIV-1-N, HIV-1-O or HIV-1-M.

22. Use according to claims 15 or 21, wherein the target is selected from an HIV antibody or antigen selected from the group consisting of p41, p120, p160, p18, p24/25, p55, p34, p40, p52, p68.

23. Use according to claim 22, wherein the HIV antigen is p160.

24. A kit for the rapid immunochromatographic detection of a target in a sample comprising the test device according to any of claims 9 to 14.

25. The kit according to claim 24, comprising further reagents and wash buffers and a manual.

26. A method for the manufacture of the test device according to any of claims 9 to 14, comprising the following steps of
(a) preparing a first colloidal gold conjugate by adding a first specific antibody or specific antigen to a conjugation buffer and then adding it to a colloidal gold solution;
(b) preparing a second colloidal gold conjugate by adding a second specific antibody or specific antigen to a conjugation buffer and then adding it to a colloidal gold solution; and
(c) preparing a first conjugate releasing site and a second conjugate releasing site by applying the first and the second colloidal gold conjugate on different pads;
wherein the first and the second conjugate releasing sites are separated by a divider or wherein the first conjugate releasing site is on the test strip and the second conjugate releasing site is within the upper site of a housing.

27. The method according to claim 26, further comprising the steps of
(d) preparing a sample application site (102), a test zone (108), a control zone (109) and a sample absorbent site (105);
(e) assembling the sample application site (102), the test zone (108), the control zone (109), the sample absorbent site (105) together with the first (103.1) and the second (103.2) conjugate releasing sites on a test strip (101);
(f) applying the first and the second colloidal gold conjugates on different sites of the same card separated by the a divider (110); and
(g) assembling the test strip (101) in a housing.

28. The method according to claim 26, further comprising the steps of
(d) preparing a sample application site (102), a test zone (108), a control zone (109) and a sample absorbent site (105);
(e) assembling the sample application site (102), the test zone (108), the control zone (109), the sample absorbent site (105) together with the first conjugate releasing site (103.1) on a test strip (101); and
(f) assembling the test strip (101) and the second conjugate releasing site (103.2) in the housing.

## Patentansprüche

1. Verfahren zur immunochromatographischen Schnelldetektion eines Targets in einer Probe, das den Schritt der Bildung eines Doppelsandwiches umfasst, durch Inkontaktbringen der Probe mit
(a) einem ersten kolloidalen Goldkonjugat, konjugiert mit einem ersten spezifischen Antikörper oder spezifischen Antigen, um das Target von einer ersten Stelle A einzufangen; gefolgt von
(b) einem zweiten spezifischen Antikörper oder spezifischen Antigen, um das Target von einer zweiten Stelle B einzufangen, wobei der zweite spezifische Antikörper oder das zweite spezifische Antigen immobilisiert ist; und gefolgt von
(c) einem zweiten kolloidalen Goldkonjugat, konjugiert mit dem zweiten spezifischen Antikörper oder spezifischen Antigen, der/das derselbe spezifische Antikörper oder dasselbe spezifische Antigen ist, wie er/es immobilisiert ist.

2. Verfahren nach Anspruch 1, das die folgenden Schritte umfasst
(d) Aufbringen der Probe auf eine Probenaufbringstelle;
(e) Erlauben, dass das Target in der Probe von der ersten Targetstelle A durch das erste kolloidale Goldkonjugat von einer ersten Konjugatfreisetzungsstelle eingefangen wird;
(f) Erlauben, dass das Target in der Probe zu einer Testzone wandert, um von der zweiten Targetstelle B durch den immobilisierten zweiten spezifischen Antikörper oder das immobilisierte zweite spezifische Antigen eingefangen zu werden;
(g) Erlauben, dass das zweite kolloidale Goldkonjugat von einer zweiten Konjugatfreisetzungsstelle freigesetzt wird, um das Target in der Probe von der zweiten Targetstelle B einzufangen;
(h) Erlauben, dass die Probe durch die Testzone und eine Kontrollzone zu einer Absorptionsstelle wandert;
(i) Erlauben, dass die ersten und die zweiten kolloidalen Goldkonjugate aus den ersten und zweiten Konjugatfreisetzungsstellen kontinuierlich freigesetzt werden, um sich zu der Testzone und der Kontrollzone auszubreiten;
(j) Nachweisen einer Farbe in der Kontrollzone; und
(k) Nachweisen einer Farbe in der Testzone.

3. Verfahren nach den Ansprüchen 1 oder 2, wobei der erste spezifische Antikörper oder das erste spezifische Antigen ausgewählt ist aus der Gruppe, bestehend aus anti-β-Choriongonadotropin-Hormon (anti-βhCG), anti-Lipoarabinomannan (LAM), Hepatitisvirus-Antikörpem gegen oder -Antigenen aus Hepatitisvirus Typ A, Hepatitisvirus Typ B oder Hepatitisvirus Typ C oder menschlichen Immunoglobulin-G-Antikörpem oder -Antigenen.

4. Verfahren nach Anspruch 3, wobei das Hepatitisvirus-Antigen Hepatitis-B-Oberflächenantigen (HBsAg) ist und der Hepatitisvirus-Antikörper anti-HBsAg ist.

5. Verfahren nach einem der Ansprüche 1 bis 4, wobei der zweite spezifische Antikörper oder das zweite spezifische Antigen ausgewählt ist aus der Gruppe, bestehend aus anti-alpha-Choriongonadotropin-Hormon (anti-αhCG), anti-Lipoarabinomannan (LAM), Hepatitisvirus-Antikörpern gegen oder -Antigenen aus Hepatitisvirus Typ A, Hepatitisvirus Typ B oder Hepatitisvirus Typ C oder menschlichen Immundefektvirus(HIV)-Antikörpern oder -Antigenen aus dem HIV-Typ HIV-1 und HIV-2 oder HIV-Untertyp HIV-1-N, HIV-1-O oder HIV-1-M.

6. Verfahren nach Anspruch 5, wobei das Hepatitisvirus-Antigen Hepatitis-B-Oberflächenantigen (HBsAg) ist, der Hepatitisvirus-Antikörpger anti-HBsAg ist und das menschliche Immundefektvirus(HIV)-Antigen HIV p160 ist.

7. Verfahren nach einem der Ansprüche 1 bis 6, wobei die Probe eine Körperflüssigkeit umfasst, die von einem Patienten erhalten ist.

8. Verfahren nach Anspruch 7, wobei die Körperflüssigkeit ausgewählt ist aus der Gruppe, bestehend aus Harn, Vollblut, Serum, Plasma und Speichel.

9. Testvorrichtung zur Durchführung des Verfahrens zur immunochromatographischen Schnelldetektion eines Targets in einer Probe nach einem der Ansprüche 1 bis 8, die ein Gehäuse umfasst, das einen Teststreifen (101) umfasst, umfassend
(a) eine Probenaufbringstelle (102);
(b) eine erste Konjugatfreisetzungsstelle (103.1), die ein erstes kolloidales Goldkonjugat umfasst, konjugiert mit einem ersten spezifischen Antikörper oder spezifischen Antigen, um das Target von einer ersten Stelle A einzufangen;
(c) eine zweite Konjugatfreisetzungsstelle (103.2), die ein zweites kolloidales Goldkonjugat umfasst, konjugiert mit einem zweiten spezifischen Antikörper oder spezifischen Antigen, um das Target von der zweiten Stelle B einzufangen;
wobei die ersten und die zweiten Konjugatfreisetzungsstellen durch einen Teiler getrennt sind oder wobei die erste Konjugatfreisetzungsstelle sich auf dem Teststreifen befindet und die zweite Konjugatfreisetzungsstelle sich auf der Oberseite des Gehäuses befindet;
(d) eine Nitrocellulose-Membran (104);
(e) eine Testzone (108), die den zweiten spezifischen Antikörper oder das zweite spezifische Antigen immobilisiert umfasst;
(f) eine Kontrollzone (109); und
(g) eine Probenabsorptionsstelle (105).

10. Testvorrichtung nach Anspruch 9, wobei der Teststreifen (101) an einer Tragerückschicht (107) mittels eines Klebers (106) befestigt ist.

11. Testvorrichtung nach Anspruch 10, wobei die Tragerückschicht (107) eine Kunststoffrückschicht ist.

12. Testvorrichtung nach Anspruch 9, 10 oder 11, wobei der zweite spezifische Antikörper oder das zweite spezifische Antigen ausgewählt ist aus der Gruppe, bestehend aus anti-alpha-Choriongonadotropin-Hormon (anti-αhCG), anti-Lipoarabinomannan (LAM), Hepatitisvirus-Antikörpem gegen oder -Antigenen aus Hepatitisvirus Typ A, Hepatitisvirus Typ B oder Hepatitisvirus Typ C oder menschlichen Immundefektvirus(HIV)-Antikörpern oder -Antigenen aus dem HIV-Typ HIV-1 und HIV-2 oder HIV-Untertyp HIV-1-N, HIV-1-O oder HIV-1-M.

13. Testvorrichtung nach Anspruch 12, wobei das Hepatitisvirus-Antigen ein Hepatitis-B-Oberflächenantigen (HBsAg) ist, der Hepatitisvirus-Antikörper anti-HBsAg ist und das menschliche Immundefektvirus(HIV)-Antigen HIV p160 ist.

14. Testvorrichtung nach einem der Ansprüche 9 bis 13, wobei die zweite Konjugatfreisetzungsstelle (103.2) in der Oberseite des Gehäuses laminiert ist.

15. Verwendung des Verfahrens nach einem der Ansprüche 1 bis 8 zum Diagnostizieren und Überwachen einer Erkrankung oder eines spezifischen Zustandes eines Patienten durch Nachweisen eines Targets in einer Probe.

16. Verwendung nach Anspruch 15, wobei der spezifische Zustand Schwangerschaft ist.

17. Verwendung nach den Ansprüchen 15 oder 16, wobei das Target menschliches Choriongonadotropin-Hormon (hCG) ist.

18. Verwendung nach Anspruch 15, wobei die Erkrankung Hepatitis ist, ausgewählt aus der Gruppe, bestehend aus Hepatitis Typ A, Hepatitis Typ B oder Hepatitis Typ C.

19. Verwendung nach Anspruch 18, wobei der ausgewählte Hepatitis-Typ Hepatitis Typ B ist.

20. Verwendung nach den Ansprüchen 15, 18 oder 19, wobei das Target Hepatitis-B-Oberflächenantigen (HBsAg) ist.

21. Verwendung nach Anspruch 15, wobei die Erkrankung eine HIV-Infektion ist, ausgewählt aus der HIV-Infektionsgruppe, bestehend aus HIV-Typ HIV-1 und HIV-2 oder HIV-Untertyp HIV-1-N, HIV-1-O oder HIV-1-M.

22. Verwendung nach den Ansprüchen 15 oder 21, wobei das Target ausgewählt ist aus einem HIV-Antikörper oder -Antigen, ausgewählt aus der Gruppe, bestehend aus p41, p120, p160, p18, p24/25, p55, p34, p40, p52, p68.

23. Verwendung nach Anspruch 22, wobei das HIV-Antigen p160 ist.

24. Kit für die immunochromatographische Schnelldetektion eines Targets in einer Probe, der die Testvorrichtung nach einem der Ansprüche 9 bis 14 umfasst.

25. Kit nach Anspruch 24, der weitere Reagentien und Waschpuffer und eine Anleitung umfasst.

26. Verfahren zur Herstellung der Testvorrichtung nach einem der Ansprüche 9 bis 14, das die folgenden Schritte umfasst
(a) Herstellen eines ersten kolloidalen Goldkonjugats durch Zugeben eines ersten spezifischen Antikörpers oder spezifischen Antigens zu einem Konjugationspuffer und anschließendes Zugeben desselben zu einer kolloidalen Goldlösung;
(b) Herstellen eines zweiten kolloidalen Goldkonjugats durch Zugeben eines zweiten spezifischen Antikörpers oder spezifischen Antigens zu einem Konjugationspuffer und anschließendes Zugeben desselben zu einer kolloidalen Goldlösung; und
(c) Herstellen einer ersten Konjugatfreisetzungsstelle und einer zweiten Konjugatfreisetzungsstelle durch Aufbringen des ersten und des zweiten kolloidalen Goldkonjugats auf unterschiedliche Kissen;
wobei die ersten und zweiten Konjugatfreisetzungsstellen durch einen Teiler getrennt sind oder wobei die erste Konjugatfreisetzungsstelle sich auf dem Teststreifen befindet und die zweite Konjugatfreisetzungsstelle sich in der Oberseite eines Gehäuses befindet.

27. Verfahren nach Anspruch 26, das weiter die Schritte umfasst
(d) Herstellen einer Probenaufbringstelle (102), einer Testzone (108), einer Kontrollzone (109) und einer Probenabsorptionsstelle (105);
(e) Zusammenfügen der Probenaufbringstelle (102), der Testzone (108), der Kontrollzone (109), der Probenabsorptionsstelle (105) mit den ersten (103.1) und den zweiten (103.2) Konjugatfreisetzungsstellen auf einem Teststreifen (101);
(f) Aufbringen der ersten und der zweiten kolloidalen Goldkonjugate auf unterschiedlichen Stellen derselben Karte, getrennt durch den Teiler (110); und
(g) Zusammenfügen des Teststreifens (101) in einem Gehäuse.

28. Verfahren nach Anspruch 26, das weiter die Schritte umfasst
(d) Herstellen einer Probenaufbringstelle (102), einer Testzone (108), einer Kontrollzone (109) und einer Probenabsorptionsstelle (105);
(e) Zusammenfügen der Probenaufbringstelle (102), der Testzone (108), der Kontrollzone (109), der Probenabsorptionsstelle (105) mit der ersten Konjugatfreisetzungsstelle (103.1) auf einem Teststreifen (101); und
(f) Zusammenfügen des Teststreifens (101) und der zweiten Konjugatfreisetzungsstelle (103.2) im Gehäuse.

## Revendications

1. Procédé de détection rapide immuno-chromatographique d'une cible dans un échantillon comprenant l'étape consistant à former un double sandwich, en mettant l'échantillon en contact avec
(a) un premier conjugué constitué d'or colloïdal conjugué à un premier anticorps spécifique ou antigène spécifique pour capturer la cible à partir d'un premier site cible A ; suivie par
(b) un deuxième anticorps spécifique ou antigène spécifique pour capturer la cible à partir d'un deuxième site cible B, dans lequel le deuxième anticorps spécifique ou antigène spécifique est immobilisé ; et suivie par
(c) un deuxième conjugué constitué d'or colloïdal conjugué au deuxième anticorps spécifique ou antigène spécifique qui est le même anticorps spécifique ou antigène spécifique que celui qui a été immobilisé.

2. Procédé selon la revendication 1, comprenant les étapes suivantes qui consistent à :
(d) appliquer l'échantillon à un site d'application de l'échantillon ;
(e) permettre à la cible dans l'échantillon d'être capturée à partir du premier site cible A par le premier conjugué constitué d'or colloïdal à partir d'un site de libération du premier conjugué ;
(f) permettre à la cible dans l'échantillon de se déplacer vers une zone d'essai pour être capturée à partir du deuxième site cible B par le deuxième anticorps spécifique ou antigène spécifique immobilisé ;
(g) permettre la libération du deuxième conjugué constitué d'or colloïdal à partir d'un site de libération du deuxième conjugué pour capturer la cible dans l'échantillon à partir du deuxième site cible B ;
(h) permettre à l'échantillon de se déplacer à travers la zone d'essai et une zone de contrôle vers un site absorbant ;
(i) permettre la libération continue des premier et deuxième conjugués constitués d'or colloïdal à partir des sites de libération des premier et deuxième conjugués pour leur propagation jusqu'à la zone d'essai et la zone de contrôle ;
(j) détecter une couleur dans la zone de contrôle ; et
(k) détecter une couleur dans la zone d'essai.

3. Procédé selon les revendications 1 ou 2, dans lequel le premier anticorps ou antigène spécifique est sélectionné dans le groupe composé d'un anticorps dirigé contre la sous-unité béta de la gonadotrophine chorionique humaine (anti-βhCG), d'un anticorps anti-Lipoarabinomannan (LAM), d'anticorps anti-hépatite contre le virus de l'hépatite A, ou d'antigènes de l'hépatite du virus de l'hépatite A, d'anticorps contre, ou d'antigènes du, virus de l'hépatite B, ou du virus de l'hépatite C ou de l'immunoglobuline G humaine.

4. Procédé selon la revendication 3, dans lequel l'antigène du virus de l'hépatite est un antigène de surface de l'hépatite B (HBsAg) et l'anticorps anti-hépatite est un anticorps anti-HBsAg.

5. Procédé selon l'une quelconque des revendications 1 à 4, dans lequel le deuxième anticorps ou antigène spécifique est sélectionné dans le groupe composé d'un anticorps dirigé contre la sous unité alpha de la gonadotrophine chorionique humaine (anti-αhCG), d'un anticorps anti-Lipoarabinomannan (LAM), d'anticorps anti-hépatite contre le virus de l'hépatite A, ou d'antigènes de l'hépatite du virus de l'hépatite A, d'anticorps contre, ou d'antigènes du, virus de l'hépatite B, ou du virus de l'hépatite C ou du virus de l'immunodéficience humaine (VIH) du VIH de type VIH-1 et VIH-2 ou de sous-type VIH-1 groupe N, VIH-1 groupe O ou VIH-1 groupe M.

6. Procédé selon la revendication 5, dans lequel l'antigène du virus de l'hépatite est un antigène de surface de l'hépatite B (HBsAg), l'anticorps anti-hépatite est l'anticorps anti-HBsAg et l'antigène du virus de l'immunodéficience humaine (VIH) est l'antigène p160 du VIH.

7. Procédé selon l'une quelconque des revendications 1 à 6, dans lequel l'échantillon comprend un fluide corporel obtenu auprès d'un sujet.

8. Procédé selon la revendication 7, dans lequel le fluide corporel est sélectionné dans le groupe composé de l'urine, de sang total, de sérum, de plasma et de salive.

9. Dispositif d'essai pour réaliser le procédé de détection rapide immuno-chromatographique d'une cible dans un échantillon de l'une quelconque des revendications 1 à 8 comprenant un coffret incluant une bande d'essai (101) comprenant
(a) un site (102) d'application de l'échantillon ;
(b) un site (103.1) de libération d'un premier conjugué comprenant un premier conjugué constitué d'or colloïdal conjugué à un premier anticorps spécifique ou antigène spécifique pour capturer la cible à partir d'un premier site cible A ;
(c) un site (103.2) de libération d'un deuxième conjugué comprenant un deuxième conjugué constitué d'or colloïdal conjugué à un deuxième anticorps spécifique ou antigène spécifique pour capturer la cible à partir du deuxième site cible B ;
dans lequel les sites de libération des premier et deuxième conjugués sont séparés par un séparateur ou dans lequel le site de libération du premier conjugué se trouve sur la bande d'essai et le site de libération du deuxième conjugué se trouve dans la face supérieure du coffret ;
(d) une membrane de nitrocellulose (104) ;
(e) une zone d'essai (108) comprenant le deuxième anticorps spécifique ou antigène spécifique immobilisé ;
(f) une zone de contrôle (109) ; et
(g) un site absorbant l'échantillon (105).

10. Dispositif d'essai selon la revendication 9, dans lequel la bande d'essai (101) est attachée à un support (107) au moyen d'un adhésif (106).

11. Dispositif d'essai selon la revendication 10, dans lequel le support (107) est un support plastique.

12. Dispositif d'essai selon la revendication 9, 10 ou 11, dans lequel le deuxième anticorps spécifique ou antigène spécifique est sélectionné dans le groupe composé d'un anticorps dirigé contre la sous-unité alpha de la gonadotrophine chorionique humaine (anti-αhCG), d'un anticorps anti-Lipoarabinomannan (LAM), d'anticorps anti-hépatite contre le virus de l'hépatite A ou d'antigènes de l'hépatite du virus de l'hépatite A, d'anticorps contre, ou d'antigènes du, virus de l'hépatite B, ou du virus de l'hépatite C ou du virus de l'immunodéficience humaine (VIH) de type VIH-1 et VIH-2 ou de sous-type VIH-1 groupe N, VIH-1 groupe O ou VIH-1 groupe M.

13. Dispositif d'essai selon la revendication 12, dans lequel l'antigène du virus de l'hépatite est un antigène de surface de l'hépatite B (HBsAg), l'anticorps anti-hépatite est l'anticorps anti-HBsAg et l'antigène du virus de l'immunodéficience humaine (VIH) est l'antigène p160 du VIH.

14. Dispositif d'essai selon l'une quelconque des revendications 9 à 13, dans lequel le site (103.2) de libération du deuxième conjugué est stratifié dans la face supérieure du coffret.

15. Utilisation du procédé selon l'une quelconque des revendications 1 à 8 pour le diagnostic et la surveillance d'une maladie ou d'un état spécifique d'un sujet en détectant une cible dans un échantillon.

16. Utilisation selon la revendication 15, dans laquelle l'état spécifique est une grossesse.

17. Utilisation selon les revendications 15 ou 16, dans laquelle la cible est l'hormone gonadotrophine chorionique humaine (hCG).

18. Utilisation selon la revendication 15, dans laquelle la maladie est une hépatite sélectionnée dans le groupe composé de l'hépatite A, de l'hépatite B ou de l'hépatite C.

19. Utilisation selon la revendication 18, dans laquelle le type de l'hépatite sélectionnée est l'hépatite B.

20. Utilisation selon les revendications 15, 18 ou 19, dans laquelle la cible est un antigène de surface de l'hépatite B (HBsAg).

21. Utilisation selon la revendication 15, dans laquelle la maladie est une infection par le VIH sélectionnée du groupe d'infections par le VIH composé du VIH de type VIH-1 et VIH-2 ou du sous-type VIH-1 groupe N, VIH-1 groupe O ou VIH-1 groupe M.

22. Utilisation selon les revendications 15 ou 21, dans laquelle la cible est sélectionnée d'un anticorps contre le VIH ou d'un antigène du VIH sélectionné du groupe composé des antigènes p41, p120, p160, p18, p24/25, p55, p34, p40, p52, p68.

23. Utilisation selon la revendication 22, dans laquelle l'antigène du VIH est l'antigène p160.

24. Trousse pour la détection rapide immuno-chromatographique d'une cible dans un échantillon comprenant le dispositif d'essai selon l'une quelconque des revendications 9 à 14.

25. Trousse selon la revendication 24, comprenant en outre des réactifs, des tampons de lavage et un manuel.

26. Procédé de fabrication du dispositif d'essai selon l'une quelconque des revendications 9 à 14, comprenant les étapes suivantes qui consistent à
(a) préparer un premier conjugué constitué d'or colloïdal en ajoutant un premier anticorps spécifique ou antigène spécifique à un tampon de conjugaison et à l'ajouter ensuite à une solution constituée d'or colloïdal ;
(b) préparer un deuxième conjugué constitué d'or colloïdal en ajoutant un deuxième anticorps spécifique ou antigène spécifique à un tampon de conjugaison et à l'ajouter ensuite à une solution constituée d'or colloïdal ; et
(c) préparer un site de libération du premier conjugué et un site de libération du deuxième conjugué en appliquant le premier et le deuxième conjugués constitués d'or colloïdal sur des tampons différents ;
dans lequel les sites de libération des premier et deuxième conjugués sont séparés par un séparateur, ou dans lequel le site de libération du premier conjugué se trouve sur la bande d'essai et le site de libération du deuxième conjugué se trouve dans le site supérieur d'un coffret.

27. Procédé selon la revendication 26, comprenant en outre les étapes qui consistent à :
(d) préparer un site (102) d'application de l'échantillon, une zone d'essai (108), une zone de contrôle (109) et un site absorbant l'échantillon (105) ;
(e) assembler le site (102) d'application de l'échantillon, la zone d'essai (108), la zone de contrôle (109), le site absorbant l'échantillon (105) ensemble avec le site (103.1) de libération du premier conjugué et le site (103.2) de libération du deuxième conjugué sur une bande d'essai (101) ;
(f) appliquer le premier et le deuxième conjugués constitués d'or colloïdal sur des sites différents de la même carte séparés par le séparateur (110) ; et
(g) assembler la bande d'essai (101) dans un coffret.

28. Procédé selon la revendication 26, comprenant en outre les étapes qui consistent à
(d) préparer un site (102) d'application de l'échantillon, une zone d'essai (108), une zone de contrôle (109) et un site absorbant l'échantillon (105) ;
(e) assembler le site (102) d'application de l'échantillon, la zone d'essai (108), la zone de contrôle (109), le site absorbant l'échantillon (105) ensemble avec le site (103.1) de libération du premier conjugué sur une bande d'essai (101) ; et
(f) assembler la bande d'essai (101) et le site (103.2) de libération du deuxième conjugué dans le coffret.
